# EUROPEAN PATENT APPLICATION

(11) **EP 2 719 359 A1**
(43) Date of publication of application: **16.04.2014**
(21) Application number: 13187936.3
(22) Date of filing: 09.10.2013
(51) Int. Cl.: A61F 2/42

(54) **Prosthesis**

(30) Priority: 09.10.2012 GB 201218081
(71) Applicant: MatOrtho Limited, Leatherhead, Surrey KT22 7BA (GB)
(72) Inventor: Tuke, Michael Anthony, Leatherhead, Surrey KT22 7BA (GB); Watson, Michael Andrew, Leatherhead, Surrey KT22 7BA (GB)
(74) Representative: Smaggasgale, Gillian Helen

(57) **Abstract**

A prosthetic ankle joint comprising: a first component having a lower articular bearing surface, the first component being configured for connection to the distal end of the tibia such that the lower articular bearing surface is remote from the tibia; a second component having an upper articular bearing surface, the second component being configured for connection to the tarsal bone such that the upper articular bearing surface is remote from the tarsal bone; a third component interposed between the first and second component, the third component having an upper bearing surface configured to be slidable on the lower bearing surface of the first component and a lower bearing surface configured to be slidable on the upper surface of the second component; wherein at least one of said components and/or said bearing surfaces being configured to allow the third component to be slidable in an anterior/posterior direction but to prevent the third component being slidable in a medial/lateral direction.

## Description

The present invention relates to a prosthesis. More particularly, it relates to a prosthetic ankle joint. Still more particularly it relates to a three-component ankle prosthesis.

The ankle joint is formed by the distal end of the tibia and the proximal end of the tarsal bone. The joint enables the angle between the top of the foot, the dorsum, and the tibia to increase and decrease, i.e. the foot to exhibit plantar-flexion and dorsiflexion,

The efficient functioning of the ankle is extremely important to the well-being and mobility of the human body. The ankle is a complex arrangement comprising three joints, the talocrucral joint which may be considered as the ankle joint proper, the subtalar joint and the inferior tibio-fibular joint. For the purposes of the present application the term ankle will refer specifically to the talo-crural joint. This joint can be regarded as a hinge joint that connects the distal end of the tibia with the proximal end of the talus. The joint is bound by a complex arrangement of several ligaments and muscles.

Diseases such as rheumatoid- and osteo-arthritis can cause damage to the joint causing pain and erosion. Bone erosion may cause the bones themselves to attempt to compensate for the erosion which may result in the bone becoming misshapen. The joint may also be damaged by accident.

Operations to replace the ankle joint with an artificial implant are well-known and widely practiced. In one arrangement the prosthesis used is a hinge. However, more sophisticated arrangements are known. In WO00/69373 a three component prosthesis is described. The first component, which is attached to the distal end of the tibia, has a spherical bearing surface. The second component, which is attached to the tarsal bone has a bearing surface which has a convex shape in the frontal plane, and concave sulcus in the frontal plane. The third component is located between the first and second components and has two surfaces which are complementary to and engage tho upper convex and the lower concave sulcus surfaces to be fully congruent with the first and second components, The third component is shaped to allow the non-fixed axis of the rotation of the articulation to be reproduced while maintaining full congruence. The bearing surfaces on the third component are shaped to be freely slidable and individually non-captive both in a sagittal plane and a frontal plane orthogonal to the sagittal plane. This arrangement also allows freedom of rotation such that twist can be achieved.

Without wishing to be bound by any theory it is believed that this ability to be freely slidable and non-captive both in the sagittal plane and a frontal plane orthogonal to the sagittal plane is advantageous. However, this freedom of movement does not mirror the natural arrangement. This is because whilst eversion and inversion of the natural ankle does occur, this is not achieved by the interaction of the ankle joint per se but rather by the action of the sub-talar joints . In addition, the rotation achieved in the prior art arrangement does not mirror the natural joint. It is therefore desirable to provide a prosthesis which offers some of the advantages of the three component system while more closely imitating the effects of the natural joint and limits the rotation twist.

Thus according to the present invention there is provided a prosthetic ankle joint comprising:
a first component having a lower articular bearing surface, the first component being configured for connection to the distal end of the tibia such that the lower articular bearing surface is remote from the tibia;
a second component having an upper articular bearing surface, the second component being configured for connection to the tarsal bone such that the upper articular bearing surface is remote from the tarsal bone;
a third component interposed between the first and second component, the third component having an upper bearing surface configured to be slidable on the lower bearing surface of the first component and a lower bearing surface configured to be slidable on the upper surface of the second component;
wherein at least one of said components and/or said bearing surfaces being configured to allow the third component to be slidable in an anterior/posterior direction but to prevent the third component being slidable in a medial/lateral direction.

By this means the prosthesis of the present invention achieves a range of motion which is more nearly replicates the natural articulation than has been achievable heretofore. With this arrangement, the non-fixed axis of rotation is reproduced while maintaining full congruence. In particular, motion in dorsiflexion and plantarflexion which replicates that of the natural ankle is achieved.

The arrangement of the present invention may be achieved by any suitable means. In one arrangement, this can be achieved by shaping of the complementary surfaces between the first and third component and/or between the second and third component.

For example, the lower bearing surface on the first component can be configured to be of partial cylindrical cross-section from front to back with a corresponding arrangement on the upper bearing surface of the third component together with a saddle shaped upper bearing surface of the third component with a corresponding arrangement on the lower bearing surface of the third component. Additionally or alternatively the first component may include one or more fingers extending downwardly from edges thereof to prevent lateral/medial sliding. These fingers may be configured to replicate one or more of the malleoli.

Additionally or alternatively one or more tracks may be provided in one or more of the bearing surfaces, said tracks running in an anterior/posterior direction. In this arrangement one or more complementary protuberances will be provided in the bearing surface that in use is in contact with the bearing surface having the track such that in use the protuberance sits within the track and moves therein as the bearing surfaces slide respectively to one another, The presence of the protuberance within the track will prevent lateral/medial movement of the bearing surfaces to one another,

The bone-facing sides of the first and third components may include elements to assist with the fixation of the prosthetic components to the bone. One or both of these sides and any elements to assist fixation may be coated with material to assist with bone ingrowth. This may be hydroxyapatite.

The components may be made of any suitable materials. In one arrangement the first and second components may be made from metal and the third component from plastics,

The present invention will now be described by way of example according to the accompanying drawings in which:
- Figure 1: is a perspective view of one arrangement of the present invention with the components illustrated expanded;
- Figure 2: is an end view of the arrangement of Figure 1;
- Figure 3: is a side view of the arrangement of Figure 1;
- Figure 4: is a perspective view of the arrangement of Figure 1 with the components illustrated connected with the third component in the anterior position; and
- Figure 5: is a perspective view of the arrangement of Figure 1 with the components illustrated connected with the third component in the posterio position.

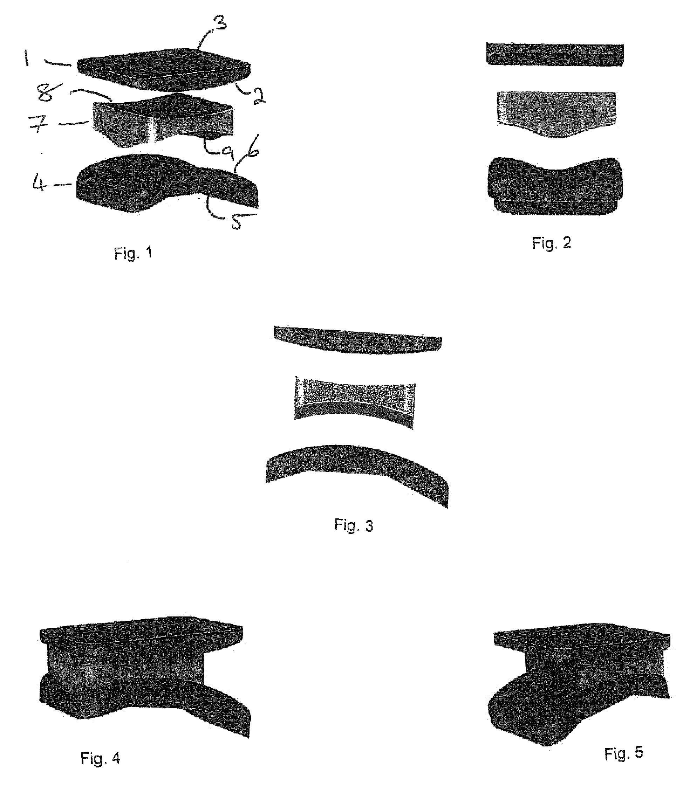

As illustrated in Figure 1, in one aspect of the present invention the prosthesis comprises three components. The first component 1 has a lower articular bearing surface 2. The first component 1 has a tibia surface 3 which is configured for connection to the distal end of the tibia. The lower articular bearing surface 2 is cylindrical from front to back as best illustrated in Figure 3. As illustrated in Figure 2, the lower bearing surface 2 does not curve from side to side. By "cylindrical" we mean that the cross-section can be taken to be a portion of a cylinder. It is therefore convex in one plane while flat in the plane at right angles to the convex plane

The second component 4 has an upper articular bearing surface 5. The second component 4 has a tarsal surface 5 which is configured for connection to the tarsal bone. The upper bearing surface 6 of the second component 4 is convex when viewed from the side. This is best illustrated in Figure 3. However, as illustrated best in Figure 2, from lateral to medial side it is concave or could be termed as being saddle shaped.

The third component 7 is interposed between the first 1 and second 4 component. The third component 7 has an upper bearing surface 8 which has a complementary shape to the lower surface 2 of component 1 such that it will be slidable on the lower bearing surface 2 of the first component 1. The third component 7 also has a lower bearing surface 9 configured to be slidable on the upper surface 6 of the second component 4. Thus the lower bearing surface 9 of the third component 7 has a curved aspect which sits within the saddle in the upper surface 6 of second component 4 and thereby prevents lateral/medial sliding.

## Claims

1. A prosthetic ankle joint comprising:
a first component having a lower articular bearing surface, the first component being configured for connection to the distal end of the tibia such that the lower articular bearing surface is remote from the tibia;
a second component having an upper articular bearing surface, the second component being configured for connection to the tarsal bone such that the upper articular bearing surface is remote from the tarsal bone;
a third component interposed between the first and second component, the third component having an upper bearing surface configured to be slidable on the lower bearing surface of the first component and a lower bearing surface configured to be slidable on the upper surface of the second component;
wherein at least one of said components and/or said bearing surfaces being configured to allow the third component to be slidable in an anterior/posterior direction but to prevent the third component being slidable in a medial/lateral direction.

2. A prosthetic ankle joint according to Claim 1 wherein the configuration which allows the third component to be slidable in an anterior/posterior direction while preventing the third component being slidable in a medial/lateral direction arrangement is shaping of the complementary surfaces between the first and third component and/or between the second and third component.

3. A prosthetic ankle joint according to Claim 1 or 2 wherein the lower bearing surface on the first component is of partial cylindrical cross-section from front to back with a corresponding arrangement on the upper bearing surface of the third component together with a saddle shaped upper bearing surface of the third component with a corresponding arrangement on the lower bearing surface of the third component.

4. A prosthetic ankle joint according to any one of Claims 1 to 3 wherein the first component includes one or more fingers extending downwardly from edges thereof to prevent lateral/medial sliding,

5. A prosthetic ankle joint according to any one of Claims 1 to 4 wherein the fingers are configured to replicate one or more of the malleoli.

6. A prosthetic ankle joint according to any one of Claims 1 to 5 wherein one or more tracks are provided in one or more of the bearing surfaces, said tracks running in an anterior/posterior direction and one or more complementary protuberances are provided in the bearing surface that in use the protuberance sits within the track and moves therein as the bearing surfaces slide respectively to one another.

7. A prosthetic ankle joint according to any one of Claims 1 to 6 wherein the bone-facing side of the first and/or third components include elements to assist with the fixation of the prosthetic components to the bone.

8. A prosthetic ankle joint according to any one of Claims 1 to 7 wherein the bone-facing sides of the first and/or third components are coated with material to assist with bone ingrowth.

9. A prosthetic ankle joint according to any one of Claims 1 to 8 wherein the first and second components may be made from metal and the third component from plastics.
